# EUROPÄISCHE PATENTSCHRIFT

(11) **EP 4 483 851 B1**
(45) Veröffentlichungstag und Bekanntmachung des Hinweises auf die Patenterteilung: **18.03.2026**
(21) Anmeldenummer: 23182230.5
(22) Anmeldetag: 28.06.2023
(51) Int. Cl.: A61F 13/15

(54) **VERFAHREN UND VORRICHTUNG ZUM DOSIEREN UND APPLIZIEREN EINER KLEINEN MENGE EINES PARTIKULÄREN MATERIALS**
METHOD AND DEVICE FOR DOSING AND APPLYING A SMALL AMOUNT OF A PARTICULATE MATERIAL
PROCÉDÉ ET DISPOSITIF DE DOSAGE ET D'APPLICATION D'UNE PETITE QUANTITÉ DE MATÉRIAU PARTICULAIRE

(43) Veröffentlichungstag der Anmeldung: 01.01.2025
(73) Patentinhaber: Paul Hartmann AG, 89522 Heidenheim (DE)
(72) Erfinder: FRÜHE, Thomas, 89518 Heidenheim (DE); FRANK, Bernd, 89555 Steinheim (DE); SPRICK, Ralf, 89564 Nattheim (DE)
(74) Vertreter: Paul Hartmann AG Patents & Licensing

(56) Entgegenhaltungen:
- EP-A2- 0 347 544
- US-A- 5 072 687

## Beschreibung

Die vorliegende Erfindung betrifft ein Verfahren zum Dosieren und Applizieren einer kleinen Menge eines partikulären Materials in einer schnelllaufenden Herstellungsmaschine für absorbierende Hygieneartikel, wobei in der Herstellungsmaschine aufeinanderfolgend Komponenten der herzustellenden Hygieneartikel als Zielstruktur für das zu applizierende partikuläre Material zugeführt und gefördert werden, wobei eine Dosierwalze mit in ihrer Außenumfangsfläche vorgesehenen Kavitäten zur Aufnahme des partikulären Materials eingesetzt wird, wobei die aufeinanderfolgend zugeführten Komponenten an und vorzugsweise unterhalb der Dosierwalze und vorzugsweise in einem Abstand zur Dosierwalze vorbeigeführt werden.

Verfahren und Vorrichtungen zum Dosieren und Applizieren von superabsorbierenden Partikeln unter Verwendung von kontinuierlich angetriebenen Dosierwalzen sind beispielsweise bekannt aus EP0347544, WO 13/0084311 A1, EP 1 655 007 A1, EP 2 583 648 A1. Hierbei werden beträchtliche Mengen superabsorbierenden Granulats zur Bildung eines SAP Laminats zwischen Flachmaterialien eingebracht. Bei einem anderen Verfahren gemäß EP 2 777 664 B1 wird ein superabsorbierendes Partikelmaterial in einen Behälter gegeben, und eine Austrittsöffnung aus dem Behälter wird mittels eines hin und her schwenkbaren keilförmigen Elements abwechselnd geöffnet und geschlossen, so dass eine jeweils möglichst gleiche und vorbestimmte Menge an Material herausrieseln und auf ein darunter laufendes Band bzw. eine dort geförderte Zielstruktur gelangen kann.

Wenn superabsorbierendes partikuläres Material in Komponenten von Hygieneartikeln appliziert wird, so handelt es sich hierbei typischerweise um eher erhebliche Mengen von wenigstens 5,0 g, insbesondere wenigstens 8,0 g, insbesondere wenigstens 10,0 g partikulären Materials. Demgegenüber befasst sich die vorliegende Erfindung mit einem Verfahren zum Dosieren und Applizieren einer kleineren Menge von beispielsweise höchstens 2,0 g oder höchstens 1,0 g partikulären Materials. Es kann sich hierbei beispielsweise um ein pH-Regulationsmittel, insbesondere eine Säure oder deren Salze oder eine Mischung davon, insbesondere Mono-Natrium-Citrat oder Di-Natrium-Citrat, handeln, welches einem eher eng begrenzten Bereich einer Absorptionskörperkomponente zugeführt und appliziert werden soll.

Typischerweise vorbekannte Vorrichtungen und Verfahren zum Dosieren und Applizieren von je Artikel größeren Mengen superabsorbierenden Materials sind nicht geeignet, um sehr geringe Mengen eines möglicherweise auch nicht optimal rieselfähigen, also kleinkörnigen oder pulverförmigen partikulären Materials in einer vorgegebenen geringen Menge zielgenau zu applizieren. Bei den bekannten Vorrichtungen und Verfahren wird partikuläres Material entweder mehr oder weniger kontinuierlich ausgegeben, oder es können nur breite Gaußverteilungen an Partikelmengen appliziert werden, was mit einer nicht konstanten Applikationsmenge und einer nicht zufriedenstellenden flächenmäßigen Verteilung oder Applikation des Materials auf der Zielstruktur einhergeht.

Wenn Dosierwalzen zum Einsatz gelangen, dann werden sie mit gleichbleibender Geschwindigkeit betrieben und zumeist gegenüber der zu beschickenden Komponente abgerollt. Ihre Kavitäten sind entweder gleichmäßig über den Außenumfang der Walze verteilt angeordnet, oder auch in Gruppen, um partikuläres Material an in einer Maschinenrichtung der Herstellungsmaschine beabstandete Komponenten zu applizieren. Dies bedeutet aber, dass je nach Erstreckung des Zielbereichs in der Maschinenrichtung und je nach Beabstandung der Komponenten in Maschinenrichtung voneinander jeweils individuell angepasste Dosierwalzen angefertigt, vorgehalten und verwendet werden müssen.

DE102021006409.7 (noch unveröffentlicht) beschreibt eine Vorrichtung und ein Verfahren zum Applizieren partikulären Materials unter Verwendung einer Dosierwalze mit Kavitäten, welche getaktet angesteuert, also dem Maschinentakt entsprechend beschleunigt und verzögert wird, um pro Takt eine vorbestimmte Anzahl an mit partikulärem Material gefüllte Kavitäten in eine Abgabeposition zu bringen. Es wurde festgestellt, dass unter bestimmten Umständen, beispielsweise bei sehr feinpudrigen Materialien, eine teilweise unvollständige Abgabe des Kavitäteninhalts erfolgen kann, wodurch eine Gefahr einer ungenaueren Dosierung entstehen kann.

Es liegt der vorliegenden Erfindung die Aufgabe zugrunde, ein Verfahren und eine Vorrichtung zum genaueren Dosieren und zielgenauen Applizieren einer kleinen Menge partikulären Materials in einer schnelllaufenden Herstellungsmaschine für absorbierende Hygieneartikel anzugeben.

Diese Aufgabe wird gelöst durch ein Verfahren der eingangs genannten Art, wobei die Dosierwalze getaktet angesteuert wird, also entsprechend der Bewegung der vorbeigeführten Komponenten beschleunigt und verzögert wird, so dass mit jedem Takt eine Anzahl von Kavitäten in eine Abgabeposition zu der gerade vorbeigeführten Komponente gebracht und darin enthaltenes partikuläres Material an die Komponente appliziert wird und wobei pro Takt die Dosierwalze um einen Drehwinkel α von mehr als 30° und weniger als 120° gedreht wird.

Es wurde erfindungsgemäß festgestellt, dass sich das Problem des genaueren Dosierens und zielgenauen Applizierens kleiner Mengen partikulären Materials während eines getakteten, also intermittierenden Betriebs der Dosierwalze durch einen derartig voreingestellten Drehwinkel α zufriedenstellend lösen lässt und dass damit auch ein wirtschaftlicher Betrieb der Herstellungsmaschine für Hygieneartikel möglich ist. Durch einen Drehwinkel α von mehr als 30° pro Takt kann die Dosierwalze bei gegebener Maschinengeschwindigkeit, also bei gegebener Geschwindigkeit der vorbeigeführten Komponenten, eine höhere Beschleunigungszeit und damit eine höhere maximale Drehgeschwindigkeit erfahren als bei einem geringeren Drehwinkel der Dosierwalze. Dabei kann das in den Kavitäten transportierte partikuläre Material auf eine höhere Geschwindigkeit beschleunigt werden und bei der nachfolgenden Verzögerung das in den Kavitäten zu der Abgabeposition transportierte partikuläre Material an der Abgabeposition leichter aus den Kavitäten freigesetzt und/oder abgeworfen werden. Hierbei wird unter einer Abgabeposition der Kavitäten der Dosierwalze verstanden, dass diese Kavitäten gegenüber der Zielstruktur exponiert sind, also nicht mehr durch Vorrichtungskomponenten, insbesondere eine ihre Entleerung verhindernde Rakeleinrichtung abgedeckt sind, so dass das darin enthaltene partikuläre Material schwerkraftunterstützt und/oder durch Zentripetalbeschleunigungen der Dosierwalze unterstützt und/oder durch die Beschleunigung und Verzögerung der Dosierwalze unterstützt ausgegeben und vorzugsweise direkt oder indirekt auf die Zielstruktur gelangt oder geleitet wird.

Durch einen solchen Drehwinkel α von mehr als 30° lässt sich bei einem getakteten und damit intermittierenden Antrieb der Dosierwalze eine vorbestimmte Menge an partikulärem Material mengen- und zielgenau und dabei ressourcen- und maschinenschonend auf eine Zielstruktur applizieren.

Bei einem sehr großen Drehwinkel pro Takt könnte die Gefahr erhöht sein, dass sich die Dosierwalze und/oder der Antrieb über Gebühr erhitzt oder erhöhter Verschleißgefahr ausgesetzt ist, insbesondere bei hoher Taktzahl in sehr schnelllaufenden Maschinen. Eine Begrenzung des Drehwinkel α nach oben kann sich daher maschinenschonend und verschleißreduzierend auswirken, wobei die vorteilhafte Wirkung auf die Dosiergenauigkeit erhalten bleibt.

Es erweist sich hierbei als vorteilhaft, wenn pro Takt die Dosierwalze um den Drehwinkel α von 35° bis 100°, insbesondere 40° bis 90°, weiter insbesondere 45° bis 60° gedreht wird.

Ein und dieselbe Dosierwalze kann für die Herstellung von Hygieneartikeln mit unterschiedlicher Menge des zu applizierenden partikulären Materials je Hygieneartikel verwendet werden. Je nach gewünschter Dosis oder Menge des zu applizierenden partikulären Materials wird ein Drehwinkel der Dosierwalze je Takt gewählt. Je größer der Drehwinkel je Takt desto mehr mit partikulärem Material gefüllte Kavitäten kommen in die Abgabeposition und werden dabei entleert. Hierdurch kann die Dosiermenge je Artikel gewissermaßen stufenlos gewählt werden. Da die Kavitäten auch sehr klein gewählt werden können, können auch geringe Mengen an partikulärem Material genau dosiert und appliziert werden.

Weiter erweist es sich als vorteilhaft, wenn das partikuläre Material mit einem Abwurfwinkel β von mindestens 0°, insbesondere größer als 0° und/oder von höchstens 90°, insbesondere von 5° bis 80°, weiter insbesondere von 10° bis 70°, weiter insbesondere von 15° bis 60° appliziert wird. Der Abwurfwinkel ist ein im radialen Querschnitt der Dosierwalze zu bestimmender, dabei ein von einer die Abgabeposition und einen Querschnittsmittelpunkt der Dosierwalze schneidenden radialen Linie und einer von dem Querschnittsmittelpunkt der Dosierwalze ausgehenden Lotrechten eingeschlossener Winkel.

Bevorzugt ist die Abgabeposition von der von dem Querschnittsmittelpunkt der Dosierwalze ausgehenden Lotrechten beabstandet und insbesondere in der Maschinenrichtung stromaufwärts zur Lotrechten der Dosierwalze verortet.

Es erweist sich hierbei als vorteilhaft, wenn die Dosierwalze in jedem Takt bis zu einer maximalen Drehgeschwindigkeit beschleunigt und um mindestens 30%, insbesondere um mindestens 50%, weiter insbesondere um mindestens 70% der maximalen Drehgeschwindigkeit verzögert, weiter insbesondere bis zum Stillstand verzögert wird. Dies ist insbesondere bei hohen Taktzahlen in schnelllaufenden Maschinen mit einer hohen maximalen Drehgeschwindigkeit der Dosierwalze verbunden, die sich auf eine vollständige Entleerung der Kavitäten vorteilhaft auswirkt bzw. dies unterstützt. Es erweist sich als vorteilhaft, dass unter Anwendung des erfindungsgemäßen Verfahrens auch partikuläres Material mit nicht ganz optimaler Rieselfähigkeit vollständig aus den Kavitäten ausgegeben und auf die Zielkomponente appliziert werden kann. Während typische superabsorbierende partikuläre Materialien eine Partikelgröße von überwiegend größer 300 µm aufweisen und als gut rieselfähig angesehen werden können, also beispielsweise wenigstens 80 Gewichtsprozent einer Partikelfraktion werden bei einer Siebrückstandsuntersuchung durch ein Sieb mit Öffnungen von 300 µm zurückgehalten, so ist dies bei demgegenüber feinerem, eher in Richtung pulverförmig gehendem partikulärem Material mitunter problematisch. Dennoch lassen sich durch den getakteten intermittierenden Antrieb der Dosierwalze auch partikuläre Materialien mit einer Partikelgröße von überwiegend kleiner 300 µm (wenigstens 80 Gewichtsprozent einer Partikelfraktion passieren bei einer Siebrückstandsuntersuchung ein Sieb mit Öffnungen von 300 µm) mit hoher Dosiergenauigkeit applizieren, indem das Material aus den Kavitäten für praktische Anwendungen rückstandslos ausgegeben wird.

Bevorzugt beträgt die maximale Drehgeschwindigkeit v der Dosierwalze 0,2 bis 3,0 m/s, insbesondere 0,3 bis 2,0 m/s, weiter insbesondere 0,5 bis 1,5 m/s. Gemeint ist hierbei eine Geschwindigkeit an der Außenumfangsfläche der Walze.

Die vorliegende Erfindung erweist sich als besonders vorteilhaft, wenn eine Maschinengeschwindigkeit von wenigstens 100 Komponenten der herzustellenden Hygieneartikeln pro Minute, insbesondere von wenigstens 200 Komponenten der herzustellenden Hygieneartikel pro Minute, insbesondere von wenigstens 300 Komponenten der herzustellenden Hygieneartikel pro Minute, insbesondere von wenigstens 400 Komponenten der herzustellenden Hygieneartikel pro Minute, insbesondere von etwa 500 Komponenten der herzustellenden Hygieneartikel pro Minute gefahren wird.

Weiter erweist sich die Erfindung als besonders vorteilhaft, wenn eine Maschinengeschwindigkeit von mehr als 500 Komponenten der herzustellenden Hygieneartikel pro Minute, insbesondere von wenigstens 1000 Komponenten der herzustellenden Hygieneartikel pro Minute, weiter insbesondere von etwa 1500 Komponenten der herzustellenden Hygieneartikel pro Minute gefahren wird. Derartige Maschinengeschwindigkeiten sind beispielsweise bei der Herstellung von kleinen Hygieneprodukten wie Inkontinenzeinlagen oder Slipeinlagen denkbar.

Insbesondere erweist es sich als vorteilhaft, wenn eine Maschinengeschwindigkeit bei der Zuführung der Komponenten von wenigstens 1 m/s, insbesondere von wenigstens 2 m/s, insbesondere von wenigstens 3 m/s, insbesondere von wenigstens 4 m/s, und weiter insbesondere von wenigstens 5 m/s gefahren wird. Dies führt dazu, dass sich eine Zielfläche der Komponente lediglich in der Größenordnung von 20-100 ms, insbesondere 40 - 60 ms und typischerweise nur 50 ms, lang in Projektion vertikal unterhalb der Dosierwalze befindet. Dementsprechend muss die getaktete Ansteuerung der Dosierwalze ausgeführt werden. Durch die hohen Taktzahlen, kurze Verweildauer der Zielfläche im Zielbereich und die hohe Maschinengeschwindigkeit kann eine höhere Drehgeschwindigkeit der Dosierwalze erreicht werden, die sich auf eine vollständige Entleerung der Kavitäten vorteilhaft auswirkt bzw. dies weiter unterstützt.

Es erweist sich weiter im Hinblick auf eine zielgenaue Positionierung und angesichts einer tangentialen Komponente des partikulären Materials beim Abgeben oder Herausschleudern aus den Kavitäten in der Abgabeposition als vorteilhaft, wenn der Abgabeposition der Kavitäten eine Leitanordnung nachgeordnet ist, mittels derer aus den Kavitäten ausgegebenes partikuläres Material in Richtung auf die Zielstruktur oder einen Zielbereich der Zielstruktur geleitet wird.

Hierbei erweist es sich als vorteilhaft, wenn die Leitanordnung eine Prall- oder Leitwand aufweist, welche zu einer horizontalen Ebene vorzugsweise einen Neigungswinkel γ zwischen 90° und 20°, insbesondere zwischen 90° und 25°, insbesondere zwischen 80° und 25°, insbesondere zwischen 60° und 25° und weiter insbesondere zwischen 50° und 25° einschließt.

Es ist auch denkbar und vorteilhaft, wenn die Prall- oder Leitwand je nach Positionierung zur Außenumfangsfläche der Dosierwalze in der Abgabeposition unterschiedliche Winkel zur horizontalen Ebene einschließt.

Alternativ kann die Prall- oder Leitwand im Querschnitt einen kurvenartigen, dabei vorzugsweise einen konvexen oder konkaven Verlauf aufweisen. Insbesondere bei einem wie nachfolgend mit Bezug zu einer erfindungsgemäßen Vorrichtung beschriebenen geringen vertikalen Abstand der an der Dosierwalze vorbeigeführten Komponenten und der Dosierwalze kann auch ein Neigungswinkel γ von weniger als 20° zwischen der Prall- oder Leitwand und einer horizontalen Ebene, insbesondere von 0° bis 10°, oder ein nur geringfügig gekrümmter Verlauf der Prall- oder Leitwand vorteilhaft sein.

Die Kavitäten bei der Dosierwalze haben die Aufgabe, partikuläres Material dosiergenau aufzunehmen und vollständig abzugeben, wenn sie in die Abgabeposition gebracht und dort insbesondere verzögert, weiter insbesondere bis zum Stillstand verzögert werden. Sie sollten also mit wohldefiniertem Aufnahmevolumen (Kavitätenvolumen) ausgebildet sein. Ferner soll eine gute Entleerbarkeit gewährleistet werden. Insbesondere im Hinblick auf diesen Aspekt erweist es sich als vorteilhaft, wenn sich nach innen verjüngende Kavitäten, insbesondere verrundet ausgebildete sich nach innen verjüngende Kavitäten, insbesondere kalottenförmig oder kuppelförmig ausgebildete Kavitäten bei der Dosierwalze vorgesehen werden. Insbesondere Kavitäten ohne innenliegende Ecken oder Kanten haben sich als vorteilhaft für eine vollständige Entleerbarkeit erwiesen.

Um eine vollständige Entleerung der Kavitäten in der Abgabeposition zu unterstützen, erweist es sich als vorteilhaft, wenn die Kavitäten in einem Übergang zu der Außenumfangsfläche der Dosierwalze von einer Wandung mit einem Öffnungswinkel größer al 0°, insbesondere von wenigstens 10°, weiter insbesondere von wenigstens 20°, weiter insbesondere von wenigstens 25°, insbesondere von wenigstens 30°, weiter insbesondere von wenigstens 35°, weiter insbesondere von wenigstens 40°, weiter insbesondere von wenigstens 45°, weiter insbesondere wenigstens 55° begrenzt werden.

Bevorzugt ist der Öffnungswinkel der Kavitäten in einer Richtung parallel zur Antriebsachse der Dosierwalze gleich groß oder größer als in einer Drehrichtung der Dosierwalze. Hierbei können die durch die im intermittierenden Betrieb durch die Beschleunigung und/oder Verzögerung auftretenden Kräfte besser zu einer vollständigen Entleerung der Kavitäten beitragen.

Es erweist sich als vorteilhaft, wenn jede der Kavitäten in der Außenumfangsfläche der Dosierwalze eine Kavitätenöffnung mit einer lichten Querschnittsfläche und eine an die Außenumfangsfläche der Dosierwalze angrenzende Mantelfläche und ein von der Mantelfläche und der lichten Querschnittsfläche gemeinsam umschlossenes Kavitätenvolumen aufweist, wobei ein Verhältnis des Kavitätenvolumens zu der lichten Querschnittsfläche bevorzugt einen Wert von 0,5 bis 3,0 mm³/mm², weiter bevorzugt 0,5 bis 2,0 mm³/mm², weiter bevorzugt 0,6 bis 1,5 mm³/mm², weiter bevorzugt 0,6 bis 1,0 mm³/mm² aufweist.

Weiter erweist es sich als vorteilhaft, wenn das Kavitätenvolumen wenigstens 6 mm³, insbesondere wenigstens 8 mm³, weiter insbesondere wenigstens 10 mm³, weiter insbesondere wenigstens 12 mm³, weiter insbesondere wenigstens 14 mm³ beträgt. Weiter erweist es sich als vorteilhaft, wenn das Kavitätenvolumen höchstens 50 mm³, insbesondere höchstens 40 mm³, weiter insbesondere höchstens 35 mm³ beträgt.

Bevorzugt weist jede der Kavitäten eine orthogonal zu der lichten Querschnittsfläche zu messende Kavitätentiefe auf, wobei ein Verhältnis des Kavitätenvolumens zu der jeweiligen Kavitätentiefe bevorzugt einen Wert von 5,0 bis 80,0 mm³/mm, weiter bevorzugt 6,0 bis 60,0 mm³/mm, weiter bevorzugt 8,0 bis 50,0 mm³/mm, weiter bevorzugt 10,0 bis 30,0 mm³/mm, weiter bevorzugt 12,0 bis 20,0 mm³/mm aufweist.

Weiter erweist es sich als vorteilhaft, wenn die Kavitätentiefe wenigstens 0,8 mm, insbesondere wenigstens 0,9 mm, weiter insbesondere wenigstens 1,0 mm, weiter insbesondere wenigstens 1,1 mm, und/oder höchstens 4,0 mm, insbesondere höchstens 3,0 mm beträgt.

Bevorzugt weist ein Verhältnis der lichten Querschnittsfläche zu der jeweiligen Kavitätentiefe einen Wert von 5,0 bis 80,0 mm²/mm, weiter bevorzugt 6,0 bis 60,0 mm²/mm, weiter bevorzugt 8,0 bis 40,0 mm²/mm, weiter bevorzugt 10,0 bis 30,0 mm²/mm, weiter bevorzugt 12,0 bis 25 mm²/mm auf.

Die lichte Querschnittsfläche der Kavitäten ist bevorzugt kreisrund, oval oder elliptisch ausgebildet.

Weiter erweist es sich als vorteilhaft, wenn eine größte Abmessung der lichten Querschnittsfläche einer Kavität in der Außenumfangsfläche der Dosierwalze wenigstens 2,5 mm, insbesondere wenigstens 3,0 mm, weiter insbesondere wenigstens 3,5 mm, und/oder höchstens 9,0 mm, insbesondere höchstens 8,5 mm, weiter insbesondere höchstens 8,0 mm beträgt.

Bevorzugt sind die Kavitäten in der Außenumfangsfläche der Dosierwalze in parallel zu einer Antriebsachse der Dosierwalze verlaufenden Reihen angeordnet.

Weiter bevorzugt sind die Kavitäten in der Außenumfangsfläche von Reihe zu Reihe in Richtung der Antriebsachse versetzt zueinander angeordnet.

In einer vorteilhaften Ausführungsform werden pro Takt 3 bis 20 Kavitäten in die Abgabeposition gebracht, insbesondere 4 bis 17, weiter insbesondere 5 bis 14.

In einer weiteren vorteilhaften Ausführungsform werden pro Takt mehr als eine Reihe Kavitäten, insbesondere 2 Reihen Kavitäten in die Abgabeposition gebracht. In einer alternativen Ausführungsform wird pro Takt genau eine Reihe Kavitäten in die Abgabeposition gebracht. Das kann sich insbesondere bei sehr hohen Maschinengeschwindigkeiten und/oder bei eher kleinen Zielbereichen als vorteilhaft erweisen.

Im Hinblick auf die Erzielung einer hohen Dosiergenauigkeit erweist es sich als vorteilhaft, dass die Kavitäten derart ausgebildet und in der Außenumfangsfläche der Dosierwalze angeordnet sind, dass sie in der Abgabeposition vollständig exponiert sind, also nicht durch eine Rakeleinrichtung oder deren Rakelschneide teilweise überdeckt und teilweise freigegeben sind. Dies kann beispielsweise sehr einfach erreicht werden, wenn die Kavitäten in der Außenumfangsfläche der Dosierwalze in parallel zu einer Antriebsachse der Dosierwalze verlaufenden Reihen angeordnet sind.

Die Kavitäten müssen aber nicht streng entsprechend einem quadratischen Muster angeordnet sein. Es kann sich auch als vorteilhaft erweisen, wenn die Kavitäten in der Außenumfangsfläche von Reihe zu Reihe in Richtung der Antriebsachse versetzt zueinander angeordnet sind. Auf diese Weise kann eine höhere Dichte an Kavitäten auf der Außenumfangsfläche realisiert werden.

Es kann sich auch als denkbar und vorteilhaft erweisen, wenn die Kavitäten in der Außenumfangsfläche der Dosierwalze in Gruppen angeordnet sind, die in Umfangsrichtung voneinander beabstandet sind. So lässt sich realisieren, dass in Umfangsrichtung zwischen diesen Gruppen ein kavitätenfreier Steg ausgebildet ist. Auf diesem Steg kann dann eine Rakelschneide zwischen zwei aufeinanderfolgenden Gruppen von Kavitäten positioniert sein und anliegen, wobei sich die eine Gruppe in der Abgabeposition und die andere Gruppe noch vor der Abgabeposition und durch die Rakelschneide abgedeckt befindet.

Es kann sich als vorteilhaft erweisen, wenn ein Außendurchmesser der Dosierwalze wenigstens 60 mm, insbesondere wenigstens 70 mm, insbesondere wenigstens 80 mm, und insbesondere höchstens 350 mm, insbesondere höchstens 320 mm, insbesondere höchstens 300 mm, insbesondere höchstens 250 mm, insbesondere höchstens 200 mm, insbesondere höchstens 150 mm aufweist.

Wie eingangs schon erwähnt, bezieht sich die vorliegende Erfindung insbesondere auf ein Verfahren, bei dem je Komponente und Takt eine geringe Menge partikulären Materials von höchstens 2,0 g, insbesondere von höchstens 1,5 g, insbesondere von höchstens 1,2 g, insbesondere von höchstens 1,0 g, insbesondere von höchstens 0,8 g appliziert wird. Insbesondere können mit dem erfindungsgemäßen Verfahren auch noch geringere Mengen an partikulärem Material wie höchstens 0,5 g, insbesondere höchstens 0,3 g, weiter insbesondere höchstens 0,2 g prozesssicherer appliziert werden.

Gegenstand der Erfindung ist weiter eine Vorrichtung zum Dosieren und Applizieren einer kleinen Menge eines partikulären Materials in einer schnelllaufenden Herstellungsmaschine für absorbierende Hygieneartikel, wobei in der Herstellungsmaschine aufeinanderfolgend Komponenten der herzustellenden Hygieneartikel als Zielstruktur für das zu applizierende partikuläre Material zugeführt und gefördert werden, mit einer Dosierwalze mit in ihrer Außenumfangsfläche vorgesehenen Kavitäten zur Aufnahme des partikulären Materials, und mit einer Rakeleinrichtung mit einer gegen die Außenumfangsfläche der Dosierwalze anliegenden Rakelschneide, wobei die aufeinanderfolgend zugeführten Komponenten an und vorzugsweise unterhalb der Dosierwalze und vorzugsweise in einem Abstand zur Dosierwalze vorbeigeführt werden, und mit einer Steuervorrichtung und Antriebsvorrichtung für die Dosierwalze, wobei die Steuer- und Antriebsvorrichtung derart ausgebildet ist, dass die Dosierwalze getaktet ansteuerbar ist, also entsprechend der Bewegung der vorbeigeführten Komponenten beschleunigt und verzögert wird, so dass mit jedem Takt eine Anzahl von Kavitäten in eine Abgabeposition zu der gerade vorbeigeführten Komponente gebracht und darin enthaltenes partikuläres Material an die Komponente appliziert wird, dadurch gekennzeichnet, dass ein Drehwinkel α der Dosierwalze pro Takt größer als 30° und kleiner als 120° ist.

Die Erfinder haben festgestellt, dass bei der Vorrichtung mit einem derartigen Drehwinkel auf vorteilhafte Weise eine vollständige Entleerung der Kavitäten und dabei ein maschinenschonender Betrieb der Herstellungsmaschine unterstützt wird.

Die Vorrichtung kann bevorzugt so ausgebildet sein, dass der Drehwinkel α der Dosierwalze pro Takt insbesondere 35° bis 100°, weiter insbesondere 40° bis 90°, weiter insbesondere 45° bis 60° beträgt.

Die Vorrichtung kann vorteilhafterweise so ausgebildet sein, dass die Dosierwalze derart getaktet ansteuerbar ist, dass sie in jedem Takt bis zu einer maximalen Drehgeschwindigkeit beschleunigt und um mindestens 30%, insbesondere um mindestens 50%, weiter insbesondere um mindestens 70% der maximalen Drehgeschwindigkeit verzögert, weiter insbesondere bis zum Stillstand verzögert wird.

Das kann sich insbesondere bei hohen Taktzahlen in schnelllaufenden Maschinen auf eine vollständige Entleerung der Kavitäten vorteilhaft auswirken.

Die Vorrichtung kann vorteilhafterweise so ausgebildet sein, dass eine Außenfläche der Dosierwalze verschleißmindernd vorbehandelt, insbesondere oberflächengehärtet und poliert, oder beschichtet, weiter insbesondere hartverchromt ist.

Bei einer oberflächengehärteten und polierten Außenfläche der Dosierwalze hat es sich als vorteilhaft erwiesen, wenn die Außenfläche nach der verschleißmindernden Vorbehandlung eine Härte nach Rockwell von 60+3 HRC aufweist.

Bei einer hartverchromten Außenfläche der dosierwalze hat es sich als vorteilhaft erwiesen, wenn die Außenfläche nach der verschleißmindernden Vorbehandlung eine Schichtdicke der Beschichtung von 50+3 µm aufweist.

Durch die verschleißmindernde Vorbehandlung kann eine Gefahr von Verschleiß, wie beispielsweise durch Reibung der Dosierwalze an anderen Vorrichtungsteilen wie der Rakeleinrichtung entstehenden Kratzern oder Materialabtrag, weiter vermindert werden, so dass eine Langlebigkeit der Dosierwalze, ein komplikationsarmer Betrieb und eine dauerhaftere Dosiergenauigkeit vorteilhaft unterstützt werden.

Es erweist sich als vorteilhaft, wenn ein Abwurfwinkel β mindestens 0° beträgt, insbesondere größer ist als 0° und/oder höchstens 90°, insbesondere 5° bis 80°, weiter insbesondere 10° bis 70°, weiter insbesondere 15° bis 60°beträgt.

Der Abwurfwinkel ist wie vorgehend mit Bezug zu dem Verfahren ausgeführt ein im Querschnitt der Dosierwalze zu bestimmender, dabei ein von einer die Abgabeposition und einen Querschnittsmittelpunkt der Dosierwalze schneidenden radialen Linie und einer von dem Querschnittsmittelpunkt der Dosierwalze ausgehenden Lotrechten eingeschlossener Winkel.

Bei einem derartigen Abwurfwinkel kann die durch den intermittierenden Betrieb der Dosierwalze bewirkte Beschleunigung das partikuläre Material auf vorteilhafte Weise in Richtung der Zielstruktur lenken und positionsgenauer applizieren. Dabei wird das partikuläre Material in Maschinenrichtung beschleunigt und kann insbesondere bei einem Abwurfwinkel von mehr als 0° unter Vermeidung übermäßiger Verwirbelung mit den vorbeigeführten Zielstrukturen vereinigt werden.

Ein insbesondere vertikaler Abstand der an der Dosierwalze vorbeigeführten Komponenten und der Dosierwalze beträgt vorteilhafterweise mehr als 0,0 cm, insbesondere 0,2 - 15,0 cm, weiter insbesondere 0,4 - 10,0 cm, weiter insbesondere 0,6 - 8,0 cm, weiter insbesondere 0,8 - 6,0 cm, weiter insbesondere 1,0 - 5,0 cm, weiter insbesondere 2,0 - 4,5 cm.

Dadurch kann das partikuläre Material auf vorteilhafte Weise über eine vorgesehene Zielfläche hinweg verteilt werden, wobei gleichzeitig in der Herstellungsmaschine platzsparend und aufgrund des relativ kurzen Weges des partikulären Materials mit höheren Geschwindigkeiten produziert werden kann.

Die Vorrichtung kann vorteilhafterweise so ausgebildet werden, dass der Abgabeposition der Kavitäten eine Leitanordnung nachgeordnet ist, mittels derer aus den Kavitäten ausgegebenes partikuläres Material in Richtung auf die Zielstruktur oder einen Zielbereich der Zielstruktur geleitet wird.

Die Vorrichtung kann vorteilhafterweise so ausgebildet werden, dass die Leitanordnung eine Prall- oder Leitwand aufweist, welche zu einer horizontalen Ebene einen Neigungswinkel γ zwischen 90° und 20°, insbesondere zwischen 90° und 25°, weitert insbesondere zwischen 80° und 25°, weiter insbesondere zwischen 60° und 25° und weiter insbesondere zwischen 50° und 25° einschließt.

Es ist auch denkbar und vorteilhaft, wenn die Prall- oder Leitwand der Vorrichtung je nach Positionierung zur Außenumfangsfläche der Dosierwalze in der Abgabeposition unterschiedliche Winkel zur horizontalen Ebene einschließt.

Alternativ kann die Prall- oder Leitwand im Querschnitt einen kurvenartigen, dabei vorzugsweise einen konvexen oder konkaven Verlauf aufweisen. Insbesondere bei einem relativ geringen vertikalen Abstand der an der Dosierwalze vorbeigeführten Komponenten und der Dosierwalze kann auch ein Neigungswinkel γ von weniger als 20° zwischen der Prall- oder Leitwand und einer horizontalen Ebene, insbesondere von 0° bis 10°, oder ein nur geringfügig gekrümmter Verlauf der Prall- oder Leitwand vorteilhaft sein.

Die Vorrichtung kann vorteilhafterweise so ausgebildet werden, dass bei der Dosierwalze sich verjüngende Kavitäten, insbesondere verrundet ausgebildete sich verjüngende Kavitäten, beispielsweise kalottenförmig oder kuppelförmig ausgebildete Kavitäten ausgebildet sind. Es wurde festgestellt, dass insbesondere Kavitäten ohne innenliegende Ecken oder Kanten sich auf vorteilhafte Weise vollständiger entleeren lassen.

Die Vorrichtung kann vorteilhafterweise so ausgebildet sein, dass die Kavitäten in einem Übergang zu der Außenumfangsfläche der Dosierwalze von einer Wandung mit einem Öffnungswinkel größer al 0°, insbesondere von wenigstens 10°, weiter insbesondere von wenigstens 20°, weiter insbesondere von wenigstens 25°, insbesondere von wenigstens 30°, weiter insbesondere von wenigstens 35°, weiter insbesondere von wenigstens 40°, weiter insbesondere von wenigstens 45°, weiter insbesondere wenigstens 55° begrenzt werden.

Bevorzugt ist der Öffnungswinkel der Kavitäten in einer Richtung parallel zu der Antriebsachse der Dosierwalze gleich groß oder größer als in einer Drehrichtung der Dosierwalze. Hierbei können die durch die im intermittierenden Betrieb durch die Beschleunigung und/oder Verzögerung auftretenden Kräfte besser zu einer vollständigen Entleerung der Kavitäten beitragen.

Weiter bevorzugt beträgt der Öffnungswinkel in der Drehrichtung der Dosierwalze höchstens 110°, insbesondere höchstens 90°, weiter insbesondere höchstens 70°.

Weiter bevorzugt beträgt der Öffnungswinkel in der Richtung parallel zu der Antriebsachse der Dosierwalze höchstens 150°, insbesondere höchstens 130°, weiter insbesondere höchstens 110°.

Die Vorrichtung kann vorteilhafterweise so ausgebildet sein, dass jede der Kavitäten in der Außenumfangsfläche der Dosierwalze eine Kavitätenöffnung mit einer lichten Querschnittsfläche und eine an die Außenumfangsfläche der Dosierwalze angrenzende Mantelfläche und ein von der Mantelfläche und der lichten Querschnittsfläche gemeinsam umschlossenes Kavitätenvolumen aufweist, wobei ein Verhältnis des Kavitätenvolumens zu der lichten Querschnittsfläche bevorzugt einen Wert von 0,5 bis 3,0 mm³/mm², weiter bevorzugt 0,5 bis 2,0 mm³/mm², weiter bevorzugt 0,6 bis 1,5 mm³/mm², weiter bevorzugt 0,6 bis 1,0 mm³/mm² aufweist.

Die Vorrichtung kann vorteilhafterweise so ausgebildet sein, dass das Kavitätenvolumen wenigstens 6 mm³, insbesondere wenigstens 8 mm³, weiter insbesondere wenigstens 10 mm³, weiter insbesondere wenigstens 12 mm³, weiter insbesondere wenigstens 14 mm³ beträgt.

Weiter kann die Vorrichtung vorteilhafterweise so ausgebildet sein, dass das Kavitätenvolumen höchstens 50 mm³, insbesondere höchstens 40 mm³, weiter insbesondere höchstens 35 mm³ beträgt.

Die Vorrichtung kann vorteilhafterweise so ausgebildet sein, dass jede der Kavitäten eine orthogonal zu der lichten Querschnittsfläche zu messende Kavitätentiefe aufweist, wobei ein Verhältnis des Kavitätenvolumens zu der jeweiligen Kavitätentiefe bevorzugt einen Wert von 5,0 bis 80,0 mm³/mm, weiter bevorzugt 6,0 bis 60,0 mm³/mm, weiter bevorzugt 8,0 bis 50,0 mm³/mm, weiter bevorzugt 10,0 bis 30,0 mm³/mm, weiter bevorzugt 12,0 bis 20,0 mm³/mm aufweist.

Die Vorrichtung kann vorteilhafterweise so ausgebildet sein, dass eine Kavitätentiefe wenigstens 0,9 mm, insbesondere wenigstens 1,0 mm, weiter insbesondere wenigstens 1,1 mm, und/oder höchstens 4,0 mm, insbesondere höchstens 3,0 mm beträgt.

Die Vorrichtung kann vorteilhafterweise so ausgebildet sein, dass ein Verhältnis der lichten Querschnittsfläche zu der jeweiligen Kavitätentiefe bevorzugt einen Wert von 5,0 bis 80,0 mm²/mm, weiter bevorzugt 6,0 bis 60,0 mm²/mm, weiter bevorzugt 8,0 bis 40,0 mm²/mm, weiter bevorzugt 10,0 bis 30,0 mm²/mm, weiter bevorzugt 12,0 bis 25 mm²/mm aufweist.

Die lichte Querschnittsfläche der Kavitäten ist bevorzugt kreisrund, oval oder elliptisch ausgebildet.

Die Vorrichtung kann vorteilhafterweise so ausgebildet sein, dass eine größte Abmessung der lichten Querschnittsfläche einer Kavität in der Außenumfangsfläche der Dosierwalze wenigstens 2,5 mm, insbesondere wenigstens 3,0 mm, weiter insbesondere wenigstens 3,5 mm, und/oder höchstens 9,0 mm, insbesondere höchstens 8,5 mm, weiter insbesondere höchstens 8,0 mm beträgt.

Die Vorrichtung kann vorteilhafterweise so ausgebildet sein, dass die Kavitäten in der Außenumfangsfläche der Dosierwalze in parallel zu einer Antriebsachse der Dosierwalze verlaufenden Reihen angeordnet sind.

Die Vorrichtung kann vorteilhafterweise so ausgebildet sein, dass die Kavitäten in der Außenumfangsfläche von Reihe zu Reihe in Richtung der Antriebsachse versetzt zueinander angeordnet sind.

Weiter kann die Vorrichtung vorteilhafterweise so ausgebildet sein, dass pro Takt 3 bis 20 Kavitäten in die Abgabeposition gebracht werden, insbesondere 4 bis 17, weiter insbesondere 5 bis 14.

In einer weiteren vorteilhaften Ausführungsform kann die Vorrichtung so ausgebildet sein, dass pro Takt mehr als eine Reihe Kavitäten, insbesondere 2 Reihen Kavitäten in die Abgabeposition gebracht werden.

Die Vorrichtung kann vorteilhafterweise so ausgebildet sein, dass die Kavitäten in der Außenumfangsfläche der Dosierwalze in Gruppen angeordnet sind, die in Umfangsrichtung voneinander beabstandet sind, so dass in Umfangsrichtung zwischen diesen Gruppen ein kavitätenfreier Stegbereich ausgebildet ist.

Die Vorrichtung kann vorteilhafterweise so ausgebildet sein, dass ein Außendurchmesser der Dosierwalze wenigstens 60 mm, insbesondere wenigstens 70 mm, insbesondere wenigstens 80 mm, und insbesondere höchstens 350 mm, insbesondere höchstens 320 mm, insbesondere höchstens 300 mm, insbesondere höchstens 250 mm, insbesondere höchstens 200 mm, insbesondere höchstens 150 mm aufweist.

Die Vorrichtung kann vorteilhafterweise so ausgebildet sein, dass je Komponente und Takt eine geringe Menge partikulären Materials von höchstens 2,0 g, insbesondere von höchstens 1,5 g, insbesondere von höchstens 1,2 g, insbesondere von höchstens 1,0 g, insbesondere von höchstens 0,8 g appliziert wird.

Insbesondere können mit der erfindungsgemäßen Vorrichtung auch noch geringere Mengen an partikulärem Material wie höchstens 0,5 g, insbesondere höchstens 0,3 g, weiter insbesondere höchstens 0,2 g prozesssicherer appliziert werden.

Gegenstand der Erfindung ist weiter eine Herstellungsmaschine für absorbierende Hygieneartikel mit einer Vorrichtung zum Dosieren und Applizieren einer kleinen Menge eines partikulären Materials, wie sie vorausgehend beschrieben und in den beigefügten Patentansprüchen beansprucht wurde.

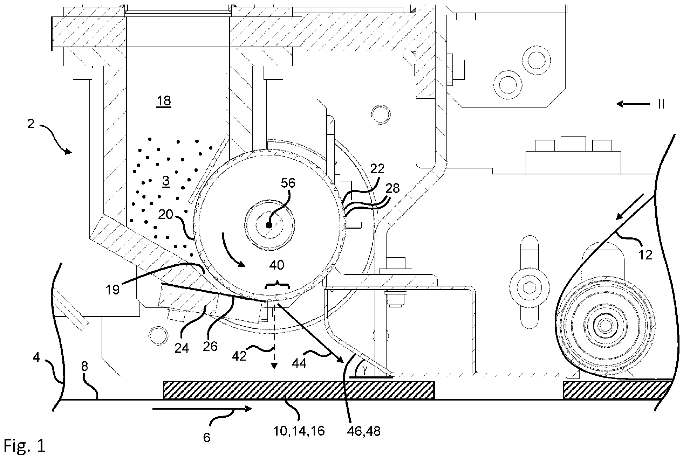

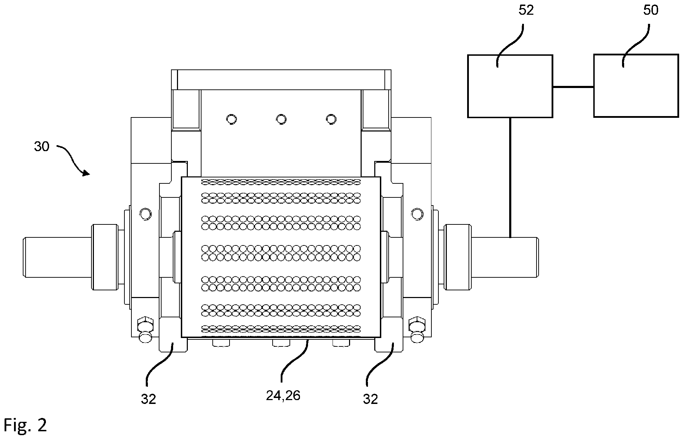

Weitere Merkmale, Einzelheiten und Vorteile der Erfindung ergeben sich aus den beigefügten Patentansprüchen und aus der beigefügten zeichnerischen Darstellung und nachfolgenden Beschreibung der Erfindung. In der Zeichnung zeigt:
Figur 1
   eine Schnittansicht durch eine erfindungsgemäße Vorrichtung zum Dosieren und Applizieren einer kleinen Menge eines partikulären Materials, mit unterhalb der Vorrichtung vorbeigeführten Komponenten eines herzustellenden Hygieneartikels und nachgeordneter Topsheetzuführung;
Figur 2
   eine Ansicht einer Dosierwalzeneinheit der Vorrichtung nach Figur 1 gesehen in Richtung des Pfeils II in Figur 1;
Figur 3
   eine perspektivische Ansicht der Dosierwalzeneinheit nach Figur 2;
Figur 4
   eine weitere perspektivische Ansicht der Dosierwalzeneinheit nach Figur 2;
Figuren 5 und 6
   verschiedene Muster der Anordnung von Kavitäten auf der Dosierwalze;

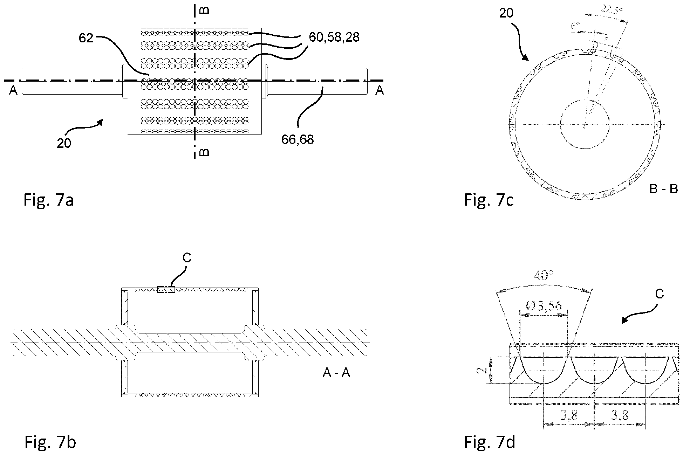

Figuren 7 a-d, 8 a-d und 9 a-e
   verschiedene Ansichten unterschiedlicher Dosierwalzen mit Antriebswelle und Einzelheiten der jeweiligen Kavitäten;
Figuren 10a-h
   weitere beispielhafte Anordnungen der Kavitäten durch eine Aufsicht in radialer Richtung auf eine jeweilige Dosierwalze;
Figuren 11a und 11b
   jeweils eine Schnittansicht durch eine Dosiereinheit mit unterschiedlichem Abwurfwinkel β.

Figur 1 zeigt eine insgesamt mit dem Bezugszeichen 2 bezeichnete Vorrichtung zum Dosieren und Applizieren einer kleinen Menge eines partikulären Materials 3, beispielsweise in Form eines pH-Regulationsmittels, in einer nur teilweise angedeuteten Herstellungsmaschine 4 für absorbierende Hygieneartikel. Von dieser Herstellungsmaschine 4 ist nur ein Teil gezeigt, in dem eine in einer Maschinenrichtung 6 endlos zugeführte Trägermaterialbahn 8, etwa zur Bildung eines Backsheets des Hygieneartikels, mit darauf in der Maschinenrichtung 6 zueinander beabstandet angeordneten Absorptionskörpern 10 zugeführt und beispielhaft und bevorzugt unterhalb der Vorrichtung 2 vorbeigeführt werden. Der Vorrichtung 2 nachgeordnet wird dann eine weitere endlose Materialbahn 12, etwa in Form eines flüssigkeitsdurchlässigen Topsheets des Hygieneartikels, zugeführt, so dass ein jeweiliger Absorptionskörper 10 sandwichartig zwischen der Trägermaterialbahn 8 und der Materialbahn 12 aufgenommen ist.

Ein jeweiliger in der Maschinenrichtung 6 zugeführter Absorptionskörper 10 bildet eine Komponente 14 eines jeweiligen herzustellenden Hygieneartikels, in welche oder an welche als Zielstruktur 16 eine bestimmte vorgegebene Menge des partikulären Materials 3 möglichst zielgenau appliziert werden soll. Hierfür umfasst die Vorrichtung 2 einen Vorratsbehälter 18 zur Aufnahme des in Figur 1 nur angedeuteten partikulären Materials 3 und eine Dosierwalze 20, welche in oder bei einer unteren Öffnung 19 des Vorratsbehälters 18 derart angebracht ist, dass sie mit einem Teil ihrer Außenumfangsfläche 22 von dem partikulären Material 3 im Inneren des Vorratsbehälters 18 beaufschlagt wird und die Öffnung 19 des Vorratsbehälters 18 im Übrigen und zusammen mit einer Rakeleinrichtung 24 bzw. deren Rakelschneide 26 im wesentlichen partikeldicht verschließt.

Die Dosierwalze 20 weist wie erwähnt eine Außenumfangsfläche 22 auf, in der eine Vielzahl von Kavitäten 28 zur Aufnahme des partikulären Materials 3 ausgebildet sind.

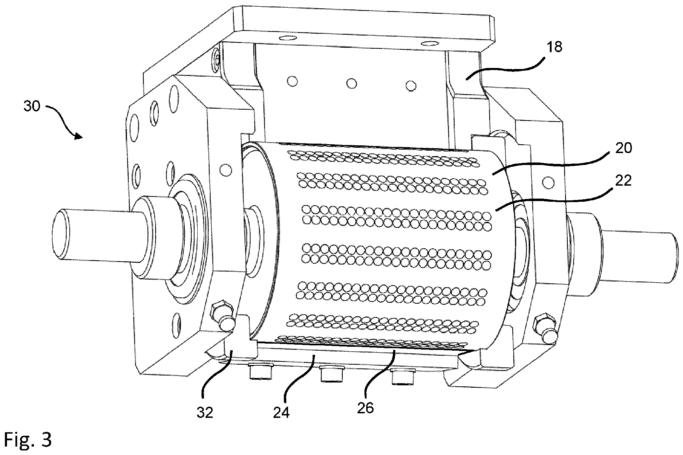

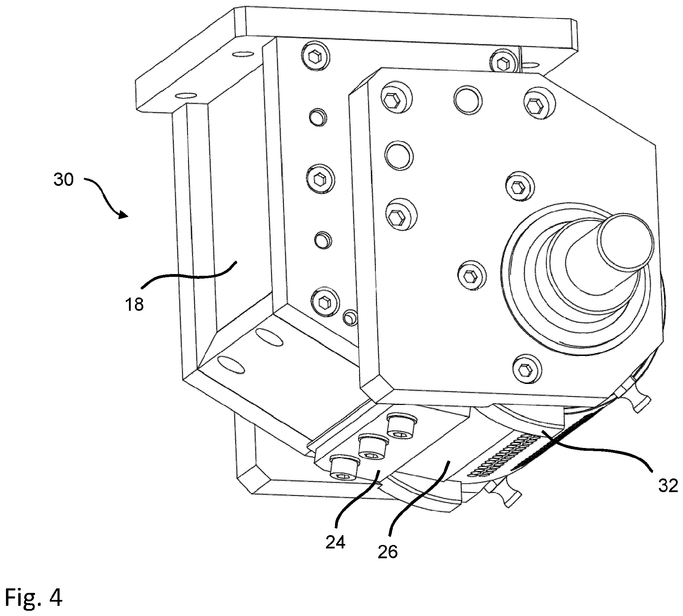

Die Figuren 2 bis 4 zeigen in weiteren Darstellungen die Vorrichtung 2 zur besseren Darstellung unter Weglassung von weiteren Komponenten der Figur 1. Man erkennt, dass der Vorratsbehälter 18 zusammen mit der darin drehbar gelagerten Dosierwalze 20 eine modular anmutende Dosiereinheit 30 bildet, die als Ganzes in der schnelllaufenden Herstellungsmaschine 4 anbringbar bzw. in die Herstellungsmaschine 4 integrierbar ist.

Wenn die Dosierwalze 20 mit ihren Kavitäten 28 in Kontakt mit dem partikulären Material 3 im Inneren des Vorratsbehälters 18 gelangt, so füllen sich die Kavitäten 28 mit diesem partikulären Material 3. Ein Austritt dieses Materials 3 nach außerhalb der Dosiereinheit 30 wird durch die bezüglich der Dosierwalze 20 unten vorgesehene Rakeleinrichtung 24 mit Ihrer Rakelschneide 26 und durch ein jeweiliges Dichtelement 32 im jeweiligen Bereich des Übergangs der Außenumfangsfläche 22 zur angrenzenden axialen Stirnseite der Dosierwalze 20 realisiert. Das Dichtelement 32 ist in vorteilhafter Weise als ein etwas nachgiebiges Kunststoffdichtelement ausgebildet und nur aus Figuren 2 bis 4 ersichtlich. Somit ist das Innere der Vorrichtung 2 bzw. ihres Vorratsbehälters 18 gegen die Umgebung und die Herstellungsmaschine 4 wirksam abgedichtet. Erst wenn jeweilige Kavitäten 28 der Dosierwalze 20 von der Rakelschneide 26 freigegeben werden, kann die darin aufgenommene geringe und genau dosierte Menge des partikulären Materials in Richtung auf die Komponente 14 des Hygieneartikels abgegeben werden. Dies kann bei der hier gezeigten Vorbeiführung der Komponenten 14 beispielhaft unterhalb und beispielhaft in einem vertikalen Abstand von 0,2-15,0 cm von der Dosierwalze 20 schwerkraftunterstützt erfolgen. Zum anderen entstehen Zentripetalbeschleunigungen infolge der noch zu erläuternden Umdrehung der Dosierwalze 20 und ferner auch bezüglich dieser Umdrehung tangentiale Komponenten bei der Abgabe des partikulären Materials 3 aus den Kavitäten 28, wenn diese in eine der Rakeleinrichtung 24 unmittelbar nachfolgende Abgabeposition 40 gedreht werden. Daher gelangt das partikuläre Material 3 nicht entlang eines gestrichelt angedeuteten Pfeils 42, also exakt in vertikaler Richtung, aus den Kavitäten 28 auf die Komponenten 14, sondern es folgt einem mehr oder weniger schräg hierzu gerichteten Weg, der lediglich rein schematisch durch einen schräg geneigten Pfeil 44 angedeutet ist. Das Material kann und wird wohl einer bogenförmigen nicht dargestellten Bahn folgen. Zur Unterstützung der zielgenauen Applikation ist auch eine Leitanordnung 46 mit einer Prall- oder Leitwand 48 vorgesehen, welche unterschiedliche Neigungswinkel γ zur Horizontalen aufweist. Beispielsweise ist der Neigungswinkel γ der Prall- oder Leitwand 48 in einem oberen Bereich nahezu 90°, und in einem unteren Bereich beträgt er eher ca. 40°. Alternativ kann die Prall- oder Leitwand auch einen kurvenartigen oder gekrümmten Verlauf aufweisen.

Erfindungsgemäß wird die Dosierwalze 20 nicht mit einer konstanten kontinuierlichen Umdrehungsgeschwindigkeit angetrieben, sondern sie wird getaktet angesteuert und hierbei entsprechend der Bewegung der vorbeigeführten Komponenten 14 beschleunigt und verzögert, dabei vorzugsweise bis zum Stillstand verzögert, so dass mit jedem Takt eine Anzahl von Kavitäten 28 in die Abgabeposition 40 in Bezug auf die gerade vorbeigeführte Komponente 14 gebracht und darin enthaltenes partikuläres Material 3 an die Komponente 14 appliziert wird. Es stellte sich heraus, dass hierdurch eine weitaus genauere Dosierung von partikulärem Material und Abgabe an eine jeweilige Komponente erreicht werden kann. Durch eine entsprechend getaktete Ansteuerung mittels einer elektronischen Steuervorrichtung 50 und einer Antriebsvorrichtung 52 (in Figur 2 schematisch angedeutet) lässt sich erreichen, dass eine jeweils vorbestimmte Anzahl von Kavitäten 28 entsprechend einem je Takt gewählten Drehwinkel für die Dosierwalze 20 in die Abgabeposition 40 gelangt. Durch die sich bei dem erfindungsgemäßen Drehwinkel α von mehr als 30° ergebenden hohen Beschleunigungen und Verzögerungen wird die vollständige Abgabe des partikulären Materials 3 aus jeder in die Abgabeposition 40 gebrachten Kavität 28 erreicht. Dies kann weiter durch eine noch zu beschreibende Formgebung der Kavitäten 28 und/oder durch Behandlung einer Außenfläche der Dosierwalze 20, insbesondere einschließlich einer Mantelfläche der Kavitäten 28 mittels Härtung oder Beschichtung, beispielsweise Hartverchromung, und/oder einer Ausbildung der die Kavitäten 28 bildenden Oberfläche der Dosierwalze 20 aus poliertem Hartmetall unterstützt werden.

Eine Begrenzung des Drehwinkel α auf weniger als 120° reduziert eine Gefahr der Überhitzung der Dosierwalze und/oder der Antriebsvorrichtung, was sich insbesondere bei schnelllaufenden Maschinen, also bei hohen Taktzahlen maschinenschonend und verschleißreduzierend auswirkt.

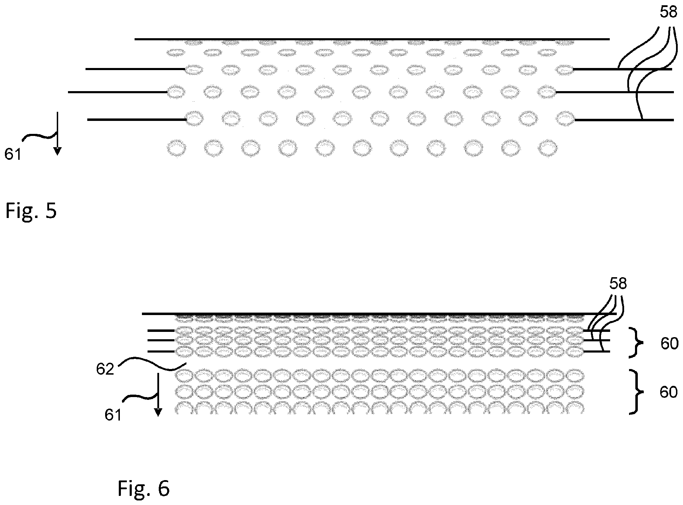

Wie in Figur 1 beispielhaft angedeutet und auch in Figuren 5 und 6 dargestellt, sind die Kavitäten 28 in zu einer geometrischen Antriebsachse 56 parallelen Reihen 58 angeordnet. Zudem erkennt man in Figuren 1 und 6, dass die Kavitäten in Gruppen 60 von jeweils beispielhaft drei Reihen 58 angeordnet sind, die in Umfangsrichtung 61 voneinander beabstandet sind. Figuren 2-4 zeigen beispielhaft Gruppen von jeweils zwei Reihen. Auf diese Weise wird zwischen den Gruppen 60 ein kavitätenfreier Stegbereich 62 gebildet, der sich für eine Anlage der Rakelschneide 26 nach einer inkrementellen Weiterbewegung der Dosierwalze 20 eignet. Es ist aber auch denkbar und vorteilhaft, wenn die Kavitäten 28 auf der Außenumfangsfläche 22 gewissermaßen gleichförmig verteilt angeordnet sind. Dabei erweist es sich als vorteilhaft, wenn die Anordnung derart ist, dass eine Rakelschneide an einer Vielzahl von inkrementellen Drehpositionen der Dosierwalze gegen die Außenumfangsfläche der Dosierwalze anliegen kann, ohne dabei eine Kavität zu überschneiden. Auf diese Weise kann dann eine definierte Anzahl von Kavitäten 28 vollständig in die Abgabeposition 40 gebracht werden.

Die Figuren 7a bis 7d zeigen die Dosierwalze 20 mit Lagerabschnitten 66 ihrer Antriebswelle 68. Sie ist vorzugsweise als Hohlwalze ausgebildet. Die Anordnung der Kavitäten 28 ist wiederum beispielhaft in Gruppen 60 von beispielhaft je zwei Reihen 58 von Kavitäten 28 ausgebildet, wobei die Gruppen 60 voneinander durch einen kavitätenfreien Stegbereich 62 beabstandet sind. In Figur 7c (Schnittansicht gemäß der Ebene B-B in Figur 7a) ist hierfür beispielhaft eine Kreissegmentteilung angegeben. Ein Drehwinkel der Dosierwalze 20 beträgt hier beispielhaft 22,5°, was 1/16 einer vollen Umdrehung der Dosierwalze entspricht.

Die Einzelheit C gemäß Figur 7d zeigt, dass die Kavitäten 28 sich in Richtung nach radial innen verjüngend ausgebildet sind. Sie sind in vorteilhafter Weise dabei verrundet ausgebildet und in ihrem radial innen liegenden Scheitelbereich kalottenförmig, insbesondere abschnittsweise kugelkalottenförmig ausgebildet. Die Kavitäten 28 weisen eine kreisrunde lichte Querschnittsfläche auf. Man erkennt weiter, dass die Kavitäten 28 in einem Übergang zu der Außenumfangsfläche der Dosierwalze von einer Wandung mit einem hier beispielhaft mit 40° angegebenen Öffnungswinkel begrenzt ist. Die Abmessungsangaben (in Millimeter) der Kavitäten in den Figuren sind aber rein beispielhaft.

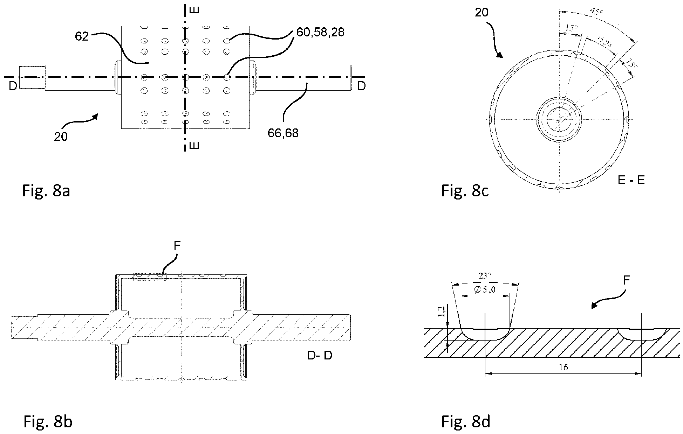

Die Figuren 8a bis 8d zeigen die Dosierwalze 20 mit Lagerabschnitten 66 ihrer Antriebswelle 68. Sie ist vorzugsweise als Hohlwalze ausgebildet. Die Anordnung der Kavitäten 28 ist wiederum beispielhaft in Gruppen 60 von beispielhaft je zwei Reihen 58 von Kavitäten 28 ausgebildet, wobei die Gruppen 60 voneinander durch einen kavitätenfreien Stegbereich 62 beabstandet sind. In Figur 8c (Schnittansicht gemäß der Ebene E-E in Figur 8a) ist hierfür beispielhaft eine Kreissegmentteilung angegeben. Ein Drehwinkel der Dosierwalze 20 beträgt hier beispielhaft 45°, was 12,5% einer vollen Umdrehung der Dosierwalze entspricht.

Die Einzelheit F gemäß Figur 8d zeigt, dass die Kavitäten 28 sich in Richtung nach radial innen verjüngend ausgebildet sind. Sie sind in vorteilhafter Weise dabei verrundet ausgebildet und in ihrem radial innen liegenden Scheitelbereich abgeflacht, insbesondere in seitlichen Abschnitten kugelkalottenförmig ausgebildet. Die Kavitäten 28 weisen eine kreisrunde lichte Querschnittsfläche auf. Man erkennt weiter, dass die Kavitäten 28 in einem Übergang zu der Außenumfangsfläche der Dosierwalze von einer Wandung mit einem hier beispielhaft mit 23° angegebenen Öffnungswinkel begrenzt ist. Die Abmessungsangaben (in Millimeter) der Kavitäten in den Figuren sind aber rein beispielhaft.

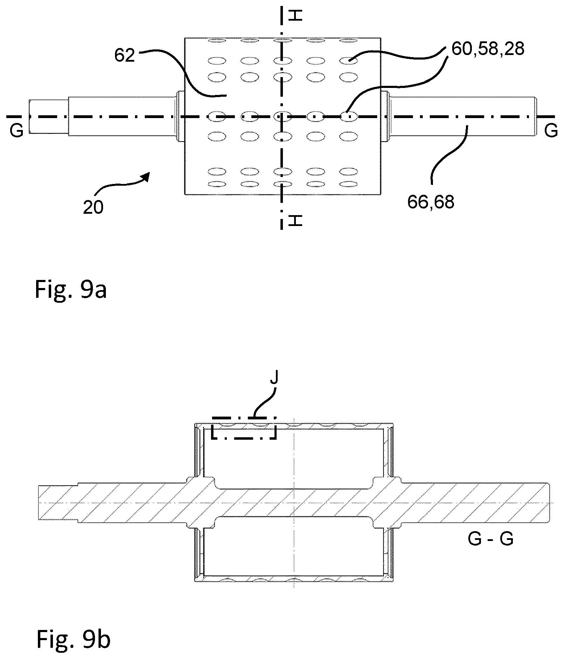

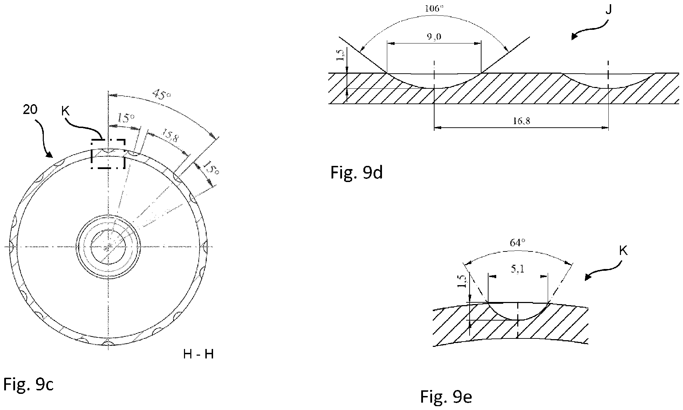

Die Figuren 9a bis 9e zeigen die Dosierwalze 20 mit Lagerabschnitten 66 ihrer Antriebswelle 68. Sie ist vorzugsweise als Hohlwalze ausgebildet. Die Anordnung der Kavitäten 28 ist wiederum beispielhaft in Gruppen 60 von beispielhaft je zwei Reihen 58 von Kavitäten 28 ausgebildet, wobei die Gruppen 60 voneinander durch einen kavitätenfreien Stegbereich 62 beabstandet sind. In Figur 9c (Schnittansicht gemäß der Ebene H-H in Figur 9a) ist hierfür beispielhaft eine Kreissegmentteilung angegeben. Ein Drehwinkel der Dosierwalze 20 beträgt hier beispielhaft 45°, was 12,5% einer vollen Umdrehung der Dosierwalze entspricht.

Die Einzelheit J gemäß Figur 9d und die Einzelheit K gemäß Figur 9e zeigt, dass die Kavitäten 28 sich in Richtung nach radial innen verjüngend ausgebildet sind. Sie sind in vorteilhafter Weise dabei verrundet ausgebildet und in ihrem radial innen liegenden Scheitelbereich kuppelförmig, dabei mit einer ovalen oder elliptischen lichten Querschnittsfläche ausgebildet. Eine Abmessung der lichten Querschnittsfläche einer Kavität in der Außenumfangsfläche der Dosierwalze weist parallel zu der Antriebswelle (vorstehend auch als Antriebsachse bezeichnet) gemessen einen anderen Wert (9,0 mm in Figur 9d), hier beispielhaft einen größeren Wert, auf als parallel zu einer Drehrichtung der Dosierwalze gemessen (5,1 mm in Figur 9e). Eine größte Abmessung der lichten Querschnittsfläche einer Kavität in der Außenumfangsfläche der Dosierwalze erstreckt sich in diesem Beispiel parallel zu der Antriebswelle. Anhand der Figur 9d erkennt man weiter, dass die Kavitäten 28 in der Richtung parallel zu der Antriebswelle in einem Übergang zu der Außenumfangsfläche der Dosierwalze von einer Wandung mit einem hier beispielhaft mit 106° angegebenen Öffnungswinkel begrenzt ist. Anhand der Figur 9e erkennt man weiter, dass die Kavitäten 28 in der Drehrichtung der Dosierwalze in einem Übergang zu der Außenumfangsfläche der Dosierwalze von einer Wandung mit einem hier beispielhaft mit 64° angegebenen Öffnungswinkel begrenzt ist. Die Abmessungsangaben (in Millimeter) der Kavitäten in den Figuren sind aber rein beispielhaft.

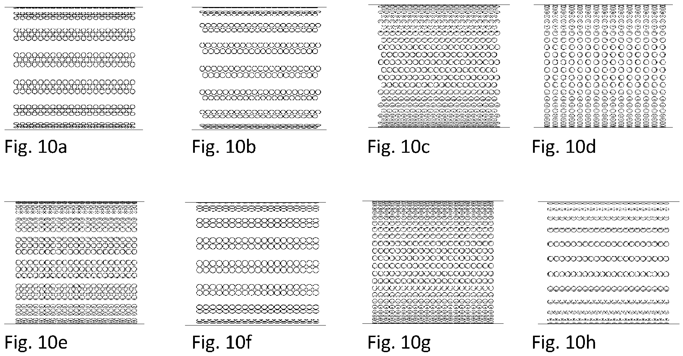

Die Figuren 10a-10h zeigen weitere beispielhafte Anordnungen der Kavitäten durch eine Aufsicht in radialer Richtung auf eine jeweilige Dosierwalze. Je dichter die Kavitäten zueinander angeordnet sind, je mehr Auftragsvolumen für partikuläres Material steht je Umdrehungstakt der Dosierwalze für die Applikation zur Verfügung. Eine geringere Anzahl an Kavitäten wie beispielsweise in Figur 8a und Figur 9a, kann hingegen eine positionsgenauere Applikation und/oder die Dosierung einer geringeren Menge an partikulärem Material und damit einen ressourcenschonenden Materialeinsatz weiter unterstützen.

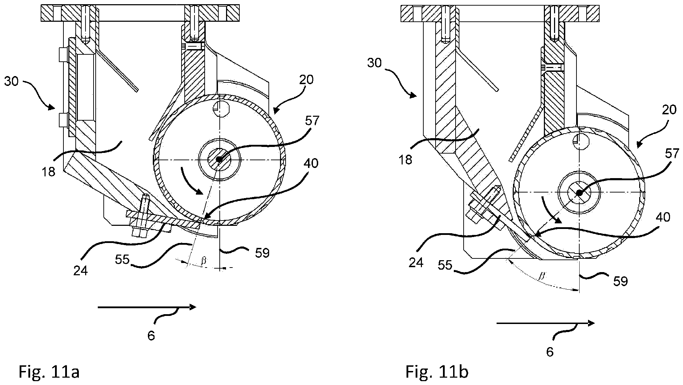

Die Figuren 11a und 11b zeigen jeweils eine Schnittansicht beispielhafter Dosiereinheiten der Vorrichtung mit unterschiedlichem Abwurfwinkel β, zur besseren Darstellung unter Weglassung von weiteren Komponenten der Figur 1. Eine einer Rakeleinrichtung 24 unmittelbar nachfolgende Abgabeposition 40 ist von der von dem Querschnittsmittelpunkt 57 der Dosierwalze 20 ausgehenden Lotrechten 59 in der Maschinenrichtung 6 stromaufwärts verortet. Der Abwurfwinkel β ist ein im radialen Querschnitt der Dosierwalze 20 zu bestimmender, dabei ein von einer die Abgabeposition 40 und den Querschnittsmittelpunkt 57 der Dosierwalze 20 schneidenden radialen Linie 55 und der von dem Querschnittsmittelpunkt 57 der Dosierwalze 20 ausgehenden Lotrechten 59 eingeschlossener Winkel. In dem in Figur 11a dargestellten Beispiel beträgt der Abwurfwinkel β 17°. In dem in Figur 11b dargestellten Beispiel beträgt der Abwurfwinkel β 47°.

## Patentansprüche

1. Verfahren zum Dosieren und Applizieren einer kleinen Menge eines partikulären Materials in einer schnelllaufenden Herstellungsmaschine (4) für absorbierende Hygieneartikel, wobei in der Herstellungsmaschine (4) aufeinanderfolgend Komponenten (14) der herzustellenden Hygieneartikel als Zielstruktur (16) für das zu applizierende partikuläre Material zugeführt und gefördert werden, wobei eine Dosierwalze (20) mit in ihrer Außenumfangsfläche (22) vorgesehenen Kavitäten (28) zur Aufnahme des partikulären Materials eingesetzt wird, wobei die aufeinanderfolgend zugeführten Komponenten (14) an und vorzugsweise unterhalb der Dosierwalze (20) und vorzugsweise in einem Abstand zur Dosierwalze (20) vorbeigeführt werden, wobei die Dosierwalze (20) getaktet angesteuert wird, also entsprechend der Bewegung der vorbeigeführten Komponenten (14) beschleunigt und verzögert wird, so dass mit jedem Takt eine Anzahl von Kavitäten (28) in eine Abgabeposition (40) zu der gerade vorbeigeführten Komponente (14) gebracht und darin enthaltenes partikuläres Material an die Komponente (14) appliziert wird, und wobei pro Takt die Dosierwalze (20) um einen Drehwinkel α von mehr als 30° und weniger als 120° gedreht wird.

2. Verfahren nach Anspruch 1 **dadurch gekennzeichnet, dass** pro Takt die Dosierwalze (20) um den Drehwinkel α von 35° bis 100°, insbesondere 40° bis 90°, weiter insbesondere 45° bis 60° gedreht wird.

3. Verfahren nach einem oder mehreren der vorstehenden Ansprüche **dadurch gekennzeichnet, dass** das partikuläre Material mit einem Abwurfwinkel β von mindestens 0°, insbesondere größer als 0° und/oder von höchstens 90°, insbesondere von 5° bis 80°, weiter insbesondere von 10° bis 70°, weiter insbesondere von 15° bis 60° appliziert wird.

4. Verfahren nach einem oder mehreren der vorstehenden Ansprüche **dadurch gekennzeichnet, dass** die Dosierwalze (20) in jedem Takt bis zu einer maximalen Drehgeschwindigkeit beschleunigt und um mindestens 30%, insbesondere um mindestens 50%, weiter insbesondere um mindestens 70% der maximalen Drehgeschwindigkeit verzögert, weiter insbesondere bis zum Stillstand verzögert wird.

5. Verfahren nach einem oder mehreren der vorstehenden Ansprüche **dadurch gekennzeichnet, dass** der Abgabeposition (40) der Kavitäten eine Leitanordnung (46) nachgeordnet ist, mittels derer aus den Kavitäten (28) ausgegebenes partikuläres Material in Richtung auf die Zielstruktur (16) oder einen Zielbereich der Zielstruktur (16) geleitet wird, wobei die Leitanordnung (46) eine Prall- oder Leitwand (48) aufweist, welche zu einer horizontalen Ebene vorzugsweise einen Neigungswinkel γ zwischen 90° und 20°, insbesondere zwischen 90° und 25°, insbesondere zwischen 80° und 25°, insbesondere zwischen 60° und 25° und weiter insbesondere zwischen 50° und 25° einschließt.

6. Vorrichtung (2) zum Dosieren und Applizieren einer kleinen Menge eines partikulären Materials in einer schnelllaufenden Herstellungsmaschine (4) für absorbierende Hygieneartikel, wobei in der Herstellungsmaschine (4) aufeinanderfolgend Komponenten (14) der herzustellenden Hygieneartikel als Zielstruktur (16) für das zu applizierende partikuläre Material zugeführt und gefördert werden, mit einer Dosierwalze (20) mit in ihrer Außenumfangsfläche (22) vorgesehenen Kavitäten (28) zur Aufnahme des partikulären Materials, und mit einer Rakeleinrichtung (24) mit einer gegen die Außenumfangsfläche (22) der Dosierwalze (20) anliegenden Rakelschneide (26), wobei die aufeinanderfolgend zugeführten Komponenten (14) an und vorzugsweise unterhalb der Dosierwalze (20) und vorzugsweise in einem Abstand zur Dosierwalze (20) vorbeigeführt werden, und mit einer Steuervorrichtung (50) und Antriebsvorrichtung (52) für die Dosierwalze (20), wobei die Steuervorrichtung (50) und Antriebsvorrichtung (52) derart ausgebildet ist, dass die Dosierwalze (20) getaktet ansteuerbar ist, also entsprechend der Bewegung der vorbeigeführten Komponenten (14) beschleunigt und verzögert wird, so dass mit jedem Takt eine Anzahl von Kavitäten (28) in eine Abgabeposition (40) zu der gerade vorbeigeführten Komponente gebracht und darin enthaltenes partikuläres Material an die Komponente (14) appliziert wird, und wobei ein Drehwinkel α der Dosierwalze pro Takt größer als 30° und kleiner als 120° ist.

7. Vorrichtung nach Anspruch 6 **dadurch gekennzeichnet, dass** der Drehwinkel α der Dosierwalze pro Takt insbesondere 35° bis 100°, weiter insbesondere 40° bis 90°, weiter insbesondere 45° bis 60° beträgt.

8. Vorrichtung nach einem oder beiden der vorstehenden Ansprüche 6 oder 7 **dadurch gekennzeichnet, dass** eine Außenfläche der Dosierwalze verschleißmindernd vorbehandelt, insbesondere oberflächengehärtet und poliert, oder beschichtet, weiter insbesondere hartverchromt ist.

9. Vorrichtung nach einem oder mehreren der vorstehenden Ansprüche 6 bis 8 **dadurch gekennzeichnet, dass** ein Abwurfwinkel β mindestens 0° beträgt, insbesondere größer ist als 0° und/oder höchstens 90°, insbesondere 5° bis 80°, weiter insbesondere 10° bis 70°, weiter insbesondere 15° bis 60° beträgt.

10. Vorrichtung nach einem oder mehreren der vorstehenden Ansprüche 6 bis 9, **dadurch gekennzeichnet, dass** ein insbesondere vertikaler Abstand der an der Dosierwalze vorbeigeführten Komponenten und der Dosierwalze mehr als 0,0 cm, insbesondere 0,2 - 15,0 cm, weiter insbesondere 0,4 - 10,0 cm, weiter insbesondere 0,6 - 8,0 cm, weiter insbesondere 0,8 - 6,0 cm, weiter insbesondere 1,0 - 5,0 cm, weiter insbesondere 2,0 - 4,5 cm beträgt.

11. Vorrichtung nach einem oder mehreren der vorstehenden Ansprüche 6 bis 10, **dadurch gekennzeichnet, dass** der Abgabeposition (40) der Kavitäten (28) eine Leitanordnung (46) nachgeordnet ist, mittels derer aus den Kavitäten (28) ausgegebenes partikuläres Material in Richtung auf die Zielstruktur (16) oder einen Zielbereich der Zielstruktur (16) geleitet wird, wobei die Leitanordnung (46) eine Prall- oder Leitwand (48) aufweist, welche zu einer horizontalen Ebene vorzugsweise einen Neigungswinkel γ zwischen 90° und 20°, insbesondere zwischen 90° und 25°, weiter insbesondere zwischen 80° und 25°, weiter insbesondere zwischen 60° und 25° und weiter insbesondere zwischen 50° und 25° einschließt.

12. Vorrichtung nach einem oder mehreren der vorstehenden Ansprüche 6 bis 11, **dadurch gekennzeichnet, dass** die Kavitäten (28) in einem Übergang zu der Außenumfangsfläche der Dosierwalze (20) von einer Wandung mit einem Öffnungswinkel größer al 0°, insbesondere von wenigstens 10°, weiter insbesondere von wenigstens 20°, weiter insbesondere von wenigstens 25°, insbesondere von wenigstens 30°, weiter insbesondere von wenigstens 35°, weiter insbesondere von wenigstens 40°, weiter insbesondere von wenigstens 45°, weiter insbesondere wenigstens 55° begrenzt werden.

13. Vorrichtung nach einem oder mehreren der vorstehenden Ansprüche **dadurch gekennzeichnet, dass** jede der Kavitäten (28) in der Außenumfangsfläche der Dosierwalze (20) eine Kavitätenöffnung mit einer lichten Querschnittsfläche und eine an die Außenumfangsfläche der Dosierwalze angrenzende Mantelfläche und ein von der Mantelfläche und der lichten Querschnittsfläche gemeinsam umschlossenes Kavitätenvolumen aufweist, wobei ein Verhältnis des Kavitätenvolumens zu der lichten Querschnittsfläche bevorzugt einen Wert von 0,5 bis 3,0 mm³/mm², weiter bevorzugt 0,5 bis 2,0 mm³/mm², weiter bevorzugt 0,6 bis 1,5 mm³/mm², weiter bevorzugt 0,6 bis 1,0 mm³/mm² aufweist.

14. Vorrichtung nach dem vorstehenden Anspruch 13 **dadurch gekennzeichnet, dass** jede der Kavitäten eine orthogonal zu der lichten Querschnittsfläche zu messende Kavitätentiefe aufweist, wobei ein Verhältnis des Kavitätenvolumens zu der jeweiligen Kavitätentiefe bevorzugt einen Wert von 5,0 bis 80,0 mm³/mm, weiter bevorzugt 6,0 bis 60,0 mm³/mm, weiter bevorzugt 8,0 bis 50,0 mm³/mm, weiter bevorzugt 10,0 bis 30,0 mm³/mm, weiter bevorzugt 12,0 bis 20,0 mm³/mm aufweist.

15. Vorrichtung nach dem vorstehenden Anspruch 14 **dadurch gekennzeichnet, dass** ein Verhältnis der lichten Querschnittsfläche zu der jeweiligen Kavitätentiefe bevorzugt einen Wert von 5,0 bis 80,0 mm²/mm, weiter bevorzugt 6,0 bis 60,0 mm²/mm, weiter bevorzugt 8,0 bis 40,0 mm²/mm, weiter bevorzugt 10,0 bis 30,0 mm²/mm, weiter bevorzugt 12,0 bis 25 mm²/mm aufweist.

## Claims

1. Method for metering and applying a small amount of a particulate material in a high-speed manufacturing machine (4) for absorbent hygiene articles, wherein components (14) of the hygiene articles to be produced are successively supplied and conveyed in the manufacturing machine (4) as a target structure (16) for the particulate material that is to be applied, wherein a metering roller (20) with cavities (28) provided in its outer circumferential surface (22) is used to receive the particulate material, wherein the successively supplied components (14) are guided past and preferably below the metering roller (20) and preferably at a distance from the metering roller (20), wherein the metering roller (20) is controlled in a clocked manner, i.e. is accelerated and decelerated according to the movement of the components (14) guided past, so that with each clock cycle a number of cavities (28) are brought into a discharge position (40) with respect to the component (14) just guided past, and particulate material contained in the cavities is applied to the component (14), and wherein the metering roller (20) is rotated by an angle of rotation α of more than 30° and less than 120° per clock cycle.

2. Method according to Claim 1, **characterized in that** the metering roller (20) is rotated, per clock cycle, by the angle of rotation α of 35° to 100°, in particular 40° to 90°, more particularly 45 to 60°.

3. Method according to one or more of the preceding claims, **characterized in that** the particulate material is applied with a throw-off angle β of at least 0°, in particular of greater than 0° and/or of at most 90°, in particular of 5° to 80°, more particularly of 10° to 70°, more particularly of 15° to 60°.

4. Method according to one or more of the preceding claims, **characterized in that** the metering roller (20) is accelerated in each clock cycle up to a maximum rotational speed and decelerated by at least 30%, in particular by at least 50%, more particularly by at least 70% of the maximum rotational speed, more particularly decelerated until it comes to a standstill.

5. Method according to one or more of the preceding claims, **characterized in that** the discharge position (40) of the cavities is followed downstream by a guide arrangement (46) by means of which particulate material discharged from the cavities (28) is guided in the direction of the target structure (16) or a target region of the target structure (16), wherein the guide arrangement (46) has a baffle or guide wall (48) which, with respect to a horizontal plane, preferably encloses an angle of inclination γ of between 90° and 20°, in particular of between 90° and 25°, in particular of between 80° and 25°, in particular of between 60° and 25°, and more particularly of between 50° and 25°.

6. Device (2) for metering and applying a small amount of a particulate material in a high-speed manufacturing machine (4) for absorbent hygiene articles, wherein components (14) of the hygiene articles to be produced are successively supplied and conveyed in the manufacturing machine (4) as a target structure (16) for the particulate material that is to be applied, having a metering roller (20) with cavities (28) provided in its outer circumferential surface (22) for the purpose of receiving the particulate material, and having a doctor device (24) with a doctor blade (26) resting against the outer circumferential surface (22) of the metering roller (20), wherein the successively supplied components (14) are guided past and preferably below the metering roller (20) and preferably at a distance from the metering roller (20), and having a control device (50) and drive device (52) for the metering roller (20), wherein the control device (50) and drive device (52) are designed in such a way that the metering roller (20) can be controlled in a clocked manner, i.e. is accelerated and decelerated according to the movement of the components (14) being guided past, so that with each clock cycle a number of cavities (28) are brought into a discharge position (40) with respect to the component just guided past, and particulate material contained in the cavities is applied to the component (14), and wherein an angle of rotation α of the metering roller is greater than 30° and less than 120° per clock cycle.

7. Device according to Claim 6, **characterized in that** the angle of rotation α of the metering roller per clock cycle is in particular 35° to 100°, more particularly 40° to 90°, more particularly 45° to 60°.

8. Device according to one or both of preceding Claims 6 and 7, **characterized in that** an outer surface of the metering roller is pre-treated to reduce wear, in particular surface-hardened and polished, or coated, more particularly hard-chrome plated.

9. Device according to one or more of preceding Claims 6 to 8, **characterized in that** a throw-off angle β is at least 0°, in particular greater than 0° and/or at most 90°, in particular 5° to 80°, more particularly 10° to 70°, more particularly 15° to 60°.

10. Device according to one or more of preceding Claims 6 to 9, **characterized in that** an in particular vertical distance between the metering roller and the components guided past the metering roller is more than 0.0 cm, in particular 0.2 - 15.0 cm, more particularly 0.4 - 10.0 cm, more particularly 0.6 - 8.0 cm, more particularly 0.8 - 6.0 cm, more particularly 1.0 - 5.0 cm, more particularly 2.0 - 4.5 cm.

11. Device according to one or more of preceding Claims 6 to 10, **characterized in that** the discharge position (40) of the cavities (28) is followed downstream by a guide arrangement (46) by means of which particulate material discharged from the cavities (28) is guided in the direction of the target structure (16) or a target region of the target structure (16), wherein the guide arrangement (46) has a baffle or guide wall (48) which, with respect to a horizontal plane, preferably encloses an angle of inclination γ of between 90° and 20°, in particular of between 90° and 25°, more particularly of between 80° and 25°, more particularly of between 60° and 25° and more particularly of between 50° and 25°.

12. Device according to one or more of preceding Claims 6 to 11, **characterized in that** the cavities (28), in a transition to the outer circumferential surface of the metering roller (20), are delimited by a wall with an opening angle greater than 0°, in particular of at least 10°, more particularly of at least 20°, more particularly of at least 25°, in particular of at least 30°, more particularly of at least 35°, more particularly of at least 40°, more particularly of at least 45°, more particularly of at least 55°.

13. Device according to one or more of the preceding claims, **characterized in that** each of the cavities (28) in the outer circumferential surface of the metering roller (20) has a cavity opening with a clear cross-sectional area and a jacket surface adjoining the outer circumferential surface of the metering roller and a cavity volume enclosed jointly by the jacket surface and the clear cross-sectional area, wherein a ratio of the cavity volume to the clear cross-sectional area preferably has a value of 0.5 to 3.0 mm³/mm², more preferably of 0.5 to 2.0 mm³/mm², more preferably of 0.6 to 1.5 mm³/mm², more preferably of 0.6 to 1.0 mm³/mm².

14. Device according to preceding Claim 13, **characterized in that** each of the cavities has a cavity depth to be measured orthogonally to the clear cross-sectional area, wherein a ratio of the cavity volume to the respective cavity depth preferably has a value of 5.0 to 80.0 mm³/mm, more preferably of 6.0 to 60.0 mm³/mm, more preferably of 8.0 to 50.0 mm³/mm, more preferably of 10.0 to 30.0 mm³/mm, more preferably of 12.0 to 20.0 mm³/mm.

15. Device according to preceding Claim 14, **characterized in that** a ratio of the clear cross-sectional area to the respective cavity depth preferably has a value of 5.0 to 80.0 mm²/mm, more preferably of 6.0 to 60.0 mm²/mm, more preferably of 8.0 to 40.0 mm²/mm, more preferably of 10.0 to 30.0 mm²/mm, more preferably of 12.0 to 25 mm²/mm.

## Revendications

1. Procédé pour doser et appliquer une petite quantité d'un matériau particulaire dans une machine de fabrication à grande vitesse (4) pour articles d'hygiène absorbants, où, dans la machine de fabrication (4), des composants (14) des articles d'hygiène à fabriquer sont amenés et transportés successivement en tant que structure cible (16) pour le matériau particulaire à appliquer, où un rouleau de dosage (20) avec des cavités prévues dans sa surface périphérique extérieure (22) (28) pour recevoir le matériau particulaire est utilisé, où les composants (14) amenés successivement passent au niveau et de préférence en dessous du rouleau de dosage (20) et de préférence à une distance du rouleau de dosage (20), où le rouleau de dosage (20) est commandé de manière cadencée, c'est-à-dire accéléré et ralenti en fonction du mouvement des composants (14) qui passent devant lui, de telle sorte qu'à chaque cycle, un nombre de cavités (28) sont mises dans une position de distribution (40) par rapport au composant (14) qui vient de passer devant et le matériau particulaire qu'elles contiennent est appliqué sur le composant (14), et où, à chaque cycle, le rouleau de dosage (20) est tourné d'un angle de rotation α supérieur à 30° et inférieur à 120°.

2. Procédé selon la revendication 1, **caractérisé en ce qu'**à chaque cycle, le rouleau de dosage (20) est tourné d'un angle de rotation α de 35° à 100°, en particulier de 40° à 90°, et plus particulièrement de 45° à 60°.

3. Procédé selon une ou plusieurs des revendications précédentes, **caractérisé en ce que** le matériau particulaire est appliqué avec un angle de déversement β d'au moins 0°, en particulier supérieur à 0° et/ou d'au plus 90°, en particulier de 5° à 80°, plus particulièrement de 10° à 70°, plus particulièrement de 15° à 60°.

4. Procédé selon une ou plusieurs des revendications précédentes, **caractérisé en ce que** le rouleau de dosage (20) est accéléré à chaque cycle jusqu'à une vitesse de rotation maximale et est ralenti d'au moins 30 %, en particulier d'au moins 50 %, plus particulièrement d'au moins 70 % de la vitesse de rotation maximale, plus particulièrement jusqu'à l'arrêt.

5. Procédé selon une ou plusieurs des revendications précédentes, **caractérisé en ce qu'**un agencement de guidage (46) est agencé en aval de la position de distribution (40) des cavités, au moyen duquel le matériau particulaire distribué à partir des cavités (28) est guidé en direction de la structure cible (16) ou d'une zone cible de la structure cible (16), où l'agencement de guidage (46) présente une paroi d'impact ou de guidage (48) qui forme, par rapport à un plan horizontal, de préférence un angle d'inclinaison γ compris entre 90° et 20°, en particulier entre 90° et 25°, en particulier entre 80° et 25°, en particulier entre 60° et 25° et plus particulièrement entre 50° et 25°.

6. Dispositif (2) pour doser et appliquer une petite quantité d'un matériau particulaire dans une machine de fabrication à grande vitesse (4) pour articles d'hygiène absorbants, où, dans la machine de fabrication (4), des composants (14) des articles d'hygiène à fabriquer sont amenés et transportés successivement en tant que structure cible (16) pour le matériau particulaire à appliquer, avec un rouleau de dosage (20) avec des cavités prévues dans sa surface périphérique extérieure (22) (28) pour recevoir le matériau particulaire, et avec un appareil de raclage (24) avec une lame de raclage (26) s'appuyant contre la surface périphérique extérieure (22) du rouleau de dosage (20), où les composants (14) amenés successivement passent au niveau et de préférence en dessous du rouleau de dosage (20) et de préférence à une distance du rouleau de dosage (20), et avec un dispositif de commande (50) et un dispositif d'entraînement (52) pour le rouleau de dosage (20), où le dispositif de commande (50) et le dispositif d'entraînement (52) sont réalisés de telle sorte que le rouleau de dosage (20) peut être commandé de manière cadencée, c'est-à-dire accéléré et ralenti en fonction du mouvement des composants (14) qui passent devant lui, de telle sorte qu'à chaque cycle, un nombre de cavités (28) sont mises dans une position de distribution (40) par rapport au composant qui vient de passer devant et le matériau particulaire qu'elles contiennent est appliqué sur le composant (14), et où un angle de rotation α du rouleau de dosage par cycle est supérieur à 30° et inférieur à 120°.

7. Dispositif selon la revendication 6, **caractérisé en ce que** l'angle de rotation α du rouleau de dosage par cycle est notamment de 35° à 100°, plus particulièrement de 40° à 90°, et plus particulièrement de 45° à 60°.

8. Dispositif selon l'une ou les deux revendications 6 ou 7 précédentes, **caractérisé en ce qu'**une surface extérieure du rouleau de dosage est prétraitée pour réduire l'usure, en particulier durcie en surface et polie, ou revêtue, et plus particulièrement chromée dur.

9. Dispositif selon une ou plusieurs des revendications 6 à 8 précédentes, **caractérisé en ce qu'**un angle de déversement β est d'au moins 0°, en particulier supérieur à 0° et/ou d'au plus 90°, en particulier de 5° à 80°, plus particulièrement de 10° à 70°, plus particulièrement de 15° à 60°.

10. Dispositif selon une ou plusieurs des revendications 6 à 9 précédentes, **caractérisé en ce qu'**une distance notamment verticale entre les composants passant devant le rouleau de dosage et le rouleau de dosage est supérieure à 0,0 cm, en particulier de 0,2 à 15,0 cm, en particulier de 0,4 à 10,0 cm, plus particulièrement de 0,6 à 8,0 cm, plus particulièrement de 0,8 à 6,0 cm, plus particulièrement de 1,0 à 5,0 cm, plus particulièrement de 2,0 à 4,5 cm.

11. Dispositif selon une ou plusieurs des revendications 6 à 10 précédentes, **caractérisé en ce qu'**un agencement de guidage (46) est agencé en aval de la position de distribution (40) des cavités (28), au moyen duquel le matériau particulaire distribué à partir des cavités (28) est guidé en direction de la structure cible (16) ou d'une zone cible de la structure cible (16), où l'agencement de guidage (46) présente une paroi d'impact ou de guidage (48) qui forme, par rapport à un plan horizontal, de préférence un angle d'inclinaison γ compris entre 90° et 20°, en particulier entre 90° et 25°, plus particulièrement entre 80° et 25°, plus particulièrement entre 60° et 25° et plus particulièrement entre 50° et 25°.

12. Dispositif selon une ou plusieurs des revendications 6 à 11 précédentes, **caractérisé en ce que** les cavités (28) sont délimitées, dans une transition vers la surface périphérique extérieure du rouleau de dosage (20), par une paroi avec un angle d'ouverture supérieur à 0°, en particulier d'au moins 10°, plus particulièrement d'au moins 20°, plus particulièrement d'au moins 25°, en particulier d'au moins 30°, plus particulièrement d'au moins 35°, plus particulièrement d'au moins 40°, plus particulièrement d'au moins 45°, plus particulièrement d'au moins 55°.

13. Dispositif selon une ou plusieurs des revendications précédentes, **caractérisé en ce que** chacune des cavités (28) dans la surface périphérique extérieure du rouleau de dosage (20) présente une ouverture de cavité avec une section transversale libre et une surface d'enveloppe adjacente à la surface périphérique extérieure du rouleau de dosage et un volume de cavité entouré conjointement par la surface d'enveloppe et la section transversale libre, où le rapport entre le volume de cavité et la section transversale libre présente de préférence une valeur de 0,5 à 3,0 mm³/mm², plus préférentiellement de 0,5 à 2,0 mm³/mm², plus préférentiellement de 0,6 à 1,5 mm³/mm², plus préférentiellement de 0,6 à 1,0 mm³/mm².

14. Dispositif selon la revendication 13 précédente, **caractérisé en ce que** chacune des cavités présente une profondeur de cavité à mesurer orthogonalement à la surface de section transversale libre, où un rapport entre le volume de cavité et la profondeur de cavité respective présente de préférence une valeur de 5,0 à 80,0 mm³/mm, plus préférentiellement de 6,0 à 60,0 mm³/mm, plus préférentiellement de 8,0 à 50,0 mm³/mm, plus préférentiellement de 10,0 à 30,0 mm³/mm, plus préférentiellement de 12,0 à 20,0 mm³/mm.

15. Dispositif selon la revendication 14 précédente, **caractérisé en ce qu'**un rapport entre la surface de section transversale libre et la profondeur de cavité respective présente de préférence une valeur de 5,0 à 80,0 mm²/mm, plus préférentiellement de 6,0 à 60,0 mm²/mm, plus préférentiellement de 8,0 à 40,0 mm²/mm, plus préférentiellement de 10,0 à 30,0 mm²/mm, plus préférentiellement de 12,0 à 25 mm²/mm.
